# EUROPEAN PATENT APPLICATION

(11) **EP 1 470 809 A2**
(43) Date of publication of application: **27.10.2004**
(21) Application number: 04251925.6
(22) Date of filing: 31.03.2004
(51) Int. Cl.: A61F 13/38

(54) **Device for applying a liquid to skin**

(30) Priority: 31.03.2003 US 403268
(71) Applicant: JOHNSON & JOHNSON CONSUMER COMPANIES, INC., Skillman, NJ 08558 (US)
(72) Inventor: Ulman, John Thomas, Woodbridge, NJ 07095 (US)
(74) Representative: Mercer, Christopher Paul

(57) **Abstract**

An applicator for liquids has a handle and at least one group of fibers comprising bicomponent fibers affixed to at least one end of the handle is disclosed. The wound fibers have a density from about 0.1 g / cm³ to about 0.8 g / cm³. The applicator is particularly useful for liquid bandage applications.

## Description

### Background Of The Invention

The invention relates generally to an applicator for applying liquids to surfaces such as human skin. More particularly, the invention relates to an applicataor comprising an elongated handle and a group of fibers affixed to at least one end of the handle. The fibrous group comprises bicomponent fibers and has a density range which enables the applicator to absorb liquid and deliver at least 40 percent of the absorbed liquid to the skin.

So-called "liquid bandages" have recently been introduced to consumers. Such liquid bandage materials typically include a cyanoacrylate monomer which, upon application to an affected part of the skin, polymerizes to provide a polymeric film. The polymeric film protects the underlying skin from undesirable environmental elements such as bacteria, dirt, water and the like. The polymeric film is water-resistant and wears off slowly over time.

In certain instances, the liquid bandage material requires an activator or initiator for initiating the desired polymerization process. In such instance, the polymerization initiator is conveniently included in that portion of an applicator which is used to apply the liquid bandage material to the skin.

Although liquid bandages provide the benefits described above, they have been viewed as somewhat expensive when compared with conventional adhesive bandages. This is due in part to the fact that currently available applicators for applying the liquid bandage material to the skin actually release to the skin only a relatively small portion of the total amount of the liquid bandage material carried by the applicator. It is important, then, that the amount of liquid bandage material delivered from the applicator to the skin be maximized to the extent possible.

One commercially available applicator for liquid bandages comprises a handle and a foam tip to which a polymerization initiator has been applied. The initiator functions, as it name suggests, to initiate polymerization of the monomeric component of the liquid bandage material. In use, a desired quantity of the liquid bandage material is dropped onto the applicator, after which the applicator is used to apply the liquid bandage material to the skin. The foam tip functions well to absorb the bandage material liquid and transfer it to the skin. Unfortunately, the process for making foam tip applicators containing the polymerization initiator is slow, as a result of which the applicators are fairly expensive.

Cotton swabs are commercially available and are typically used for cleaning ears or delivering actives to the skin. Existing cotton swabs are not entirely suitable for delivering liquid bandages to the skin because the swabs tend to retain liquid, thus reducing the amount of liquid which is delivered to the skin. In addition, it is thought that certain initiators may be substantive to the cotton in the swab. In this circumstance, the liquid bandage material ultimately applied to the skin may not have a sufficient amount of initiator to effectively initiate the desired polymerization of the monomeric component of the liquid bandage material.

European Patent Application No. 0363533 A1 discloses an applicator that is useful for applying liquid medicine to skin. The applicator comprises a handle and at least one group of fibers attached to the handle. The group of fibers contains at least some bicomponent fibers. The bi-component fibers are made from a first polymer and a second polymer. The first polymer and the second polymer have melting points that differ by at least 20°C. The group of fibers is heated to a temperature where the lower melting point polymer melts, but the higher melting point polymer does not melt. This enables the lower melting point polymer to flow and bond together the fibers in the group of fibers. The resulting bonded group of fibers is resistant to fraying.

Despite the disclosure of the reference, there is a continuing need for an applicator for applying a liquid to skin, where the applicator absorbs the liquid to be applied to the skin and transfers as much as possible of the absorbed liquid to the skin during the application procedure.

### Summary Of The Invention

The present invention provides an improved applicator for applying liquids to surfaces such as the skin. The applicator comprises a handle having a first end and a second end; and a group of fibers affixed to at least one of said first and second ends of the handle. The group of fibers comprises bicomponent fibers and has a density of from about 0.2 g / cm³ to about 0.8 g / cm³.

### Brief Description of the Drawings

The invention will be more clearly understood by reference to the accompanying drawings in which:
FIG. 1 is an elevational view of an applicator in accordance with the present invention;
FIG. 2 is an elevational view of a handle for use in the practice of the present invention;
FIG. 3 is a cross-section, greatly enlarged, of a bicomponent fiber having a sheath/core configuration; and
FIG. 4 is a cross-section, greatly enlarged, of a bicomponent fiber having a side-by-side configuration.

### Detailed Description Of The Invention

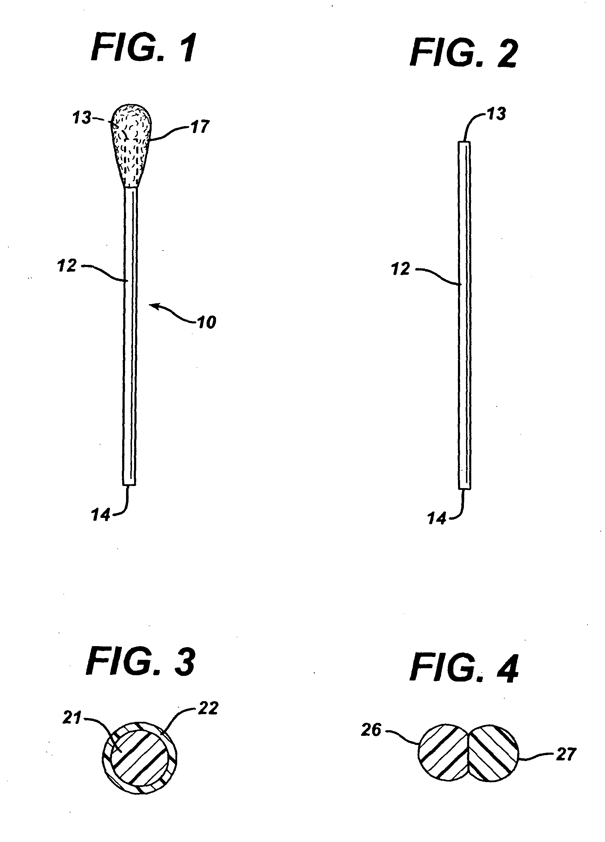

The present invention provides an applicator for liquids. Referring to FIG. 1, applicator 10 includes a handle 12 having a first end 13 and a second end 14. The shape of the handle is not critical. Typically, the handle is in the shape of a somewhat elongated rod or bar. The length of the handle may range from about 2.5 cm to about 15 cm, preferably from about 5 cm to about 10 cm. The handle can have any desired cross-section, e.g. circular, oval, square or the like. When the handle is circular in cross-section, its diameter may range from about 1 mm to about 5 mm, preferably from about 2 mm to about 4 mm. The handle may be made from any suitable material. Suitable materials include, but are not limited to, plastic, paper, wood, natural rubber, and synthetic rubber. The handle may be hollow if desired. The end of the handle to which the group of fibers will be affixed can be roughened or provided with a barbed surface to enhance securement thereto of the fibers. Adhesives may be used for this purpose as well.

The applicator of the invention comprises at least one group of fibers 17 comprising bi-component fibers affixed to at least one of the ends, e.g. end 13, of the handle. Bicomponent fibers are well known in the art and are readily commercially available. Bicomponent fibers comprise at least two different polymers. The bicomponent fibers may have a sheath/core configuration, wherein one of its polymeric components forms the core and the other polymeric component forms the sheath surrounding the core. This sheath core configuration is illustrated in FIG. 3 wherein the bicomponent fiber is indicated in cross-section by reference numeral 20, the core polymer is indicated by numeral 21 and the sheath polymer is indicated by numeral 22. In sheath/core type bicomponent fibers, the melting point of the sheath polymer should be lower than the melting point of the core polymer. Suitable sheath/core bicomponent fibers include high density polyethylene/polyethylene terephthalate sheath/core fibers; polyethylene terephthalate copolymer/polyethylene terephthalate sheath/core fibers; and the bicomponent fibers disclosed generally in EP 0 363 533 A1.

The bicomponent fibers may have a side-by-side configuration if desired. A bicomponent fiber 25 having such a configuration is shown in cross-section in FIG. 4, where numeral 26 indicates one polymeric component and numeral 27 indicates a different polymeric components. One of the two polymeric components should have a lower melting point than the other.

The configuration of the bicomponent fibers used in the invention is not critical. Other bicomponent fiber configurations known in the art may be utilized, if desired. Sheath/core type bicomponent fibers are well suited for practice of the present invention.

As indicated earlier, bicomponent fibers are commercially available. Suitable bicomponent fibers may be made from polyolefins, polyesters, polyamides and combinations thereof. Suitable polyolefins include, but are not limited to, polyethylene and polypropylene. Suitable polyesters include, but are not limited to, polyethylene terephthalate and polybutylene terephthalate. Suitable polyamides include nylon 6 and nylon 6,6. The group of fibers 17 may be formed by blending the bicomponent fibers with other fibers. Fibers useful for blending with the bi-component fibers include, but are not limited to, cotton and rayon as well as synthetic fibers such as polyolefins and polyamides. The amount of bicomponent fibers in the blend forming a group of fibers may range from about 10 percent by weight to about 100 percent by weight, based on the total weight of fibers.

The denier of the bicomponent fibers utilized in this invention may range from about 1 to about 15, preferably from about 1.5 to about 10, more preferably from about 2 to about 4. The length of the fibers may range from about 0.5 cm to about 5 cm, preferably from about 1 cm to about 4 cm, more preferably from about 2 cm to about 3.5 cm. Prior to being affixed to the handle of the applicator, the short length fibers are typically converted into a light weight web by carding or air laying techniques well known in the art. Narrow width webs, e.g. those having a width of about 40 cm, are gathered into "ropes" or "coils" prior to being fed to known winding equipment which removes a portion of the incoming rope or coil and applies it to the end of the handle while the handle is rotating about its longitudinal axis. The fibrous coils suitably have a basis weight of from about 10 grains/yard to about 25 grains/yard, preferably from about 15 grains/yard to about 21 grains/yard.

The portion of fibrous material removed from the coil is affixed to at least one end of the handle or, if desired, to both ends of the handle. For paper and wood handles, an adhesive is typically applied to the area of the handle where the fibers are to be located. The end of the fibrous coil is contacted with the adhesive coated handle and the handle is rotated about its longitudinal axis to take up the desired amount of fibers. For plastic handles, the end of the handle is typically abraded to create barbs. The end of the fibrous coil is brought into contact with the barbs and the handle is rotated to take up the desired amount of fibers. The amount of fiber web used will depend on various factors, including the amount of liquid uptake desired. The ends of the applicator are shaped into a rounded or teardrop configuration using a known heatable forming device. The fibers are heated while disposed in the forming device to bond the fibers together, thus forming group of fibers 17.

Tension is applied during the winding of the fiber web onto the handle. The amount of tension will affect the density of the resulting group of fibers. The ability of a group of fibers to absorb liquid and to transfer the absorbed liquid to the application site, e.g., the skin, depends on the density. The density of the group of fibers should range from about 0.2 g / cm³ to about 0.8 g / cm³, preferably from about 0.25 g / cm³ to about 0.6 g /cm³, more preferably from about 0.3 g / cm³ to about 0.5 g / cm³.

Applicators in accordance with the teachings of the present invention are particularly useful for applying the aforementioned liquid bandage materials to the skin. The liquid bandage material, which typically contains a cyanoacrylate monomer (e.g. iso-octyl cyanoacrylate) and minor amounts of other ingredients such as colorants, stabilizers, and the like, is applied to the applicator. The amount of liquid bandage material applied to the applicator will vary depending, e.g., on the area of the skin to be covered. The group of fibers comprising the applicator may, if necessary or desired, include an initiator to initiate polymerization of the monomeric component of the liquid bandage material. A polymerization initiator may be included in the group of fibers by first preparing a solution of the desired initiator in a suitable solvent; dipping the group of fibers in the solution; allowing excess solution to drain off; and removing the solvent to thereby leave the initiator behind. Solvent can be removed by evaporation, heating in a stream of warm air, etc. Alternatively, the initiator solution may be sprayed onto the group of fibers and dried.

The following examples are provided for illustrative purposes. The invention should not be construed as being limited to the details thereof.

### Example 1

Bicomponent fibers comprising a high density polyethylene sheath and a polyethylene terephthalate core ("PE/PET Bi"), all 2.54 cm (1 inch) in length, were purchased from Intercontinental Polymer Inc. of Tennessee. In some instances, the group of fibers 17 was made entirely from PE/PET Bi fibers. In other instances, the group of fibers 17 was made from 80% by weight PE/PET Bi fibers and 20% by weight polyethylene terephthalate fibers ("Blend").

Coils of fibrous material having a basis weight of about 20.5 grains/yard were wound around the abraded end of 7.5 cm long, 2.5 mm diameter plastic handles using known winding equipment to provide applicators in accordance with the present invention. Each group of fibers weighed approximately 0.038 g. The fiber groups covered about 1.2 cm of the end of the handle. As can be seen in Table 1, applicators were made having groups of fibers having densities ranging from about 0.37 g/cm³ to about 0.48 g/cm^{3.} Density was determined by dividing the weight of a group of fibers by its volume. Volume was determined optically using a Profile Projector Model No. PH-3500 available from Mitutoyo (Japan). The groups of fibers, i.e., the fiber tips were heat bonded at the temperatures shown in Table 1. The group of fibers in each of the several applicators was dipped in a solution comprising 1% by weight of an initiator for initiating the polymerization of iso-octyl cyanoacrylate and then dried. Four drops of BAND-AID® Brand Liquid Bandage (the monomeric component of which is iso-octyl cyanoacrylate) were applied to the group of fibers in the applicators under test. The liquid bandage material was then transferred to a 3 inch x 4 inch sheet of pre-weighed filter paper by wiping the fibrous tip of the applicator back and forth along the length of the paper until, by visual observation, no additional liquid was transferred from the applicator to the paper. Light hand pressure was used during the wiping procedure. The amount of liquid bandage material transferred to the paper was determined by weighing the liquid-wetted paper and subtracting the weight of the pre-weighed paper. Applicators that transferred to the filter paper 40% or more of the liquid which had been applied to them were considered acceptable. Applicators that transferred to the filter paper 70% or more of the liquid which had been applied to them to the paper were preferred. The results are shown in Table 1.

**Table 1**

| Sample °C | Composition | Bonding Temperature Fiber group | Density of g/cm³ | % Delivered |
|---|---|---|---|---|
| 1 | Cotton Swab Control | NA | 0.17 | 19 |
| 2 | Blend | 250 | 0.37 | 61 |
| 3 | Blend | 250 | 0.43 | 60 |
| 4 | Blend | 275 | 0.42 | 52 |
| 5 | Blend | 300 | 0.44 | 50 |
| 6 | Blend | 300 | 0.46 | 48 |
| 7 | 100% PE/PET Bi (3 denier) | 250 | 0.43 | 81 |
| 8 | 100% PE/PET Bi (3 denier) | 250 | 0.42 | 73 |
| 9 | 100% PE/PET Bi (3 denier) | 300 | 0.48 | 69 |
| 10 | 100% PE/PET Bi (3 denier) | 275 | 0.45 | 63 |
| 11 | 100% PE/PET Bi (3 denier) | 300 | 0.38 | 60 |

The Blend contained 80% bicomponent fibers with a denier of 3 and 20% polyethylene terephthalate fibers with a denier of 10. Temperature refers to the temperature in °C at which the groups of fibers were heat-bonded. NA = not applicable. % Delivered = percent of liquid bandage material transferred from the group of fibers in the applicator to the test paper.

It will be understood by those skilled in the art that a group of fibers can be applied to each of the ends 13,14 of handle 12 illustrated in FIG. 2. Further, it will be understood the applicators according to the present invention can be used for applying other liquids, e.g., antibacterial solutions, cleansing agents or medicaments to the skin. Still further it will be recognized that the inventive applicators can be used to apply liquids or semi-solids to surfaces other than skin. For example, the inventive applicator could be used to apply cleaning solvents to a variety of items requiring cleaning.

## Claims

1. An applicator comprising:
a handle having a first end and a second end; and
a group of fibers affixed to at least one of said first and second ends,
said group of fibers comprising bicomponent fibers and having a density of from 0.2 g / cm³ to 0.8 g / cm³.

2. An applicator according to Claim 1 wherein said group of fibers comprises at least 10% by weight of bicomponent fibers.

3. An applicator according to Claim 1 or Claim 2 wherein said group of fibers carries a polymerization initiator.

4. An applicator according to Claim 3 wherein said polymerization initiator is an initiator for the polymerization of an α-cyanoacrylate monomer.
